# EUROPEAN PATENT APPLICATION

(11) **EP 1 010 685 A1**
(43) Date of publication of application: **21.06.2000**
(21) Application number: 98811229.8
(22) Date of filing: 15.12.1998
(51) Int. Cl.: C07C 49/86, C07C 17/269, C07C 45/46, C07C 45/00, C07C 45/51, C07C 41/50, C07D 317/16, C11B 9/00, A61K 7/46

(54) **New tetrahydronaphthalenes**

(71) Applicant: Givaudan Roure (International) S.A., 1214 Vernier-Genève (CH)
(72) Inventor: Gygax, Peter Dr., 8117 Fällanden (CH); Gonzenbach, Hans-Ulrich Dr., 1202 Genève (CH)
(74) Representative: Patentanwälte Schaad, Balass, Menzl & Partner AG

(57) **Abstract**

The compounds of formula I are useful as fixateur in fragrance compositions. In formula I, R¹ is hydrogen or methyl and R² is alkyl.

## Description

The present invention relates to new tetrahydronaphthalenes, a process for preparing these compounds and to the use thereof.

Fragrance compositions consist of a number of components having different volatility. During storage and application more volatile compounds evaporate faster, altering the quality of the composition. To maintain the fragrant quality of such compositions over a longer period measures have to be taken to prevent early evaporation of the more volatile components. Generally, so called "fixateurs" are added to the compositions. The fixateur compounds form loose complexes with the more volatile components of the fragrance composition, thus reducing volatility thereof. The fixateur compounds may either be odorless or have an odor which contributes to the scent of the fragrance composition. For the perfumer it is easier to use odorless fixateurs, as these do not impart an additional odor and therefore allow a dosage over a wide range without altering much the fragrance quality. Further odorless fixateurs can be used in a wide range of different fragrance compositions.

It has now been found that compounds of the formula I 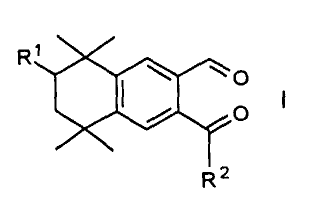 wherein R¹ is hydrogen or methyl and R² is alkyl, especially C₁₋₁₂-alkyl and preferably C₁₋₄-alkyl are odorless compounds which are useful as fixateurs.

As the skilled person knows, o-diacylarenes can not be prepared by double Friedel-Crafts-acylation of the parent arene due to the strong desactivation of the first introduced acyl group.

In principle o-acylbenzaldehydes could be prepared from o-acylcinnamic acids, however, the oxidation steps required produce wastes of transition metals (*E. Berner, Acta Chemica Scand. Ser. B, 729 (1982)*). A further possibility would be to start from an acetylheptratriene, which is not easily available and would call for a low yield air-oxidation in the last step, a procedure which is potentially dangerous (*M. Senguel, J. Chem. Soc. Perkin Trans. I, 2071 (1997)*).

Therefore a new method was needed which avoids the drawbacks of the known method for the preparation of the new compounds of formula I.

It has been found that the following 5 step method using readily available starting materials provides the desired compound of formula I with a good yield:

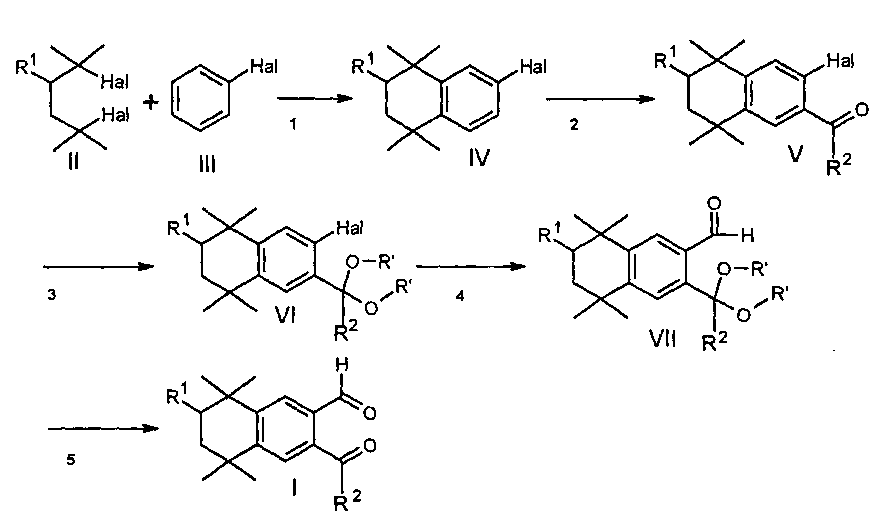

In the formulae R¹ is either hydrogen or methyl, Hal stands for halogen, especially Br or Cl, R' is alkyl, preferably lower alkyl , especially methyl or R'-R' can also stand for a divalent alkyl residue, thus forming a dioxolane ring, and R² is alkyl, especially C₁₋₁₂-alkyl and preferably C₁₋₄-alkyl.

In step 1 a halobenzene, preferably bromo- or chlorobenzene, is condensed with a 2,5-dihalo-2,5-dimethylhexane. The condensation is run in the presence of a small amount of Lewis-acid catalysts such as for example aluminium chloride, ferric chloride, bismuth chloride, to name only a few of the many catalysts capable of carrying out alkylations of arenes. The substituted tetraline derivatives IV obtained in this way can be isolated and purified. The introduction of the acyl group (step 2) is done by the reaction of the tetraline IV with an activated acid derivative in the presence af a Friedel-Crafts-acylation-catalyst. As activated acid derivatives may be used for example acid chlorides or acid anhydrides. The catalysts again may be Lewis acids as cited above. To make the procedure more economic, steps 1 and 2 can also be carried out without isolation of IV. To avoid undesired side reactions, the amount of catalyst in step 1 has to be kept small and only just before the acylation-step 2 more catalyst is added. As the acyl group of V is not compatible with the Grignard reaction (step 4), it is protected as a ketal. This can be done by reacting V with alcohols or orthoesters or by transketalisation, usually catalysed by acids such as Lewis or protic acids. Examples for acid catalysts are sulfonic acids, sulfuric acid, clays etc. Instead of monohydric alcohols diols such as for example ethylene glycol may also be used. Compounds VII are then transformed into an organometallic species such as lithium or magnesium derivatives by treatment of VI with magnesium, lithium or lithiumalkyls such as for example butyllithium. The use of magnesium is preferred for economical reasons. The formyl group is preferably introduced by reaction of the organometallic intermediate with formic acid or a derivative thereof, most easily and economically by means of formamides such as dimethylformamide, N-alkyl-formanilides or formylmorpholine. Although it is possible to isolate the formyl-ketals VII, it is more practical and more economic to hydrolyse them without any workup by treating the reaction mixture with water at an acidic pH-value.

The compounds of formula I are odorless and show excellent fixateur qualities in different fragrance compositions resulting in improved linear behaviour, i.e. high stability of the fragrance quality. They can be used for a wide range of fragrance compositions.

The following examples will illustrate the invention without limitation.

### Example 1

### 1,1,4,4-Tetramethyl-6-acetyl-7-formyl-1,2,3,4-tetrahydronaphthalene

219,6 g 2,5-Dichloro-2,2,5,5-tetramethyl-hexane dissolved in 480 ml dichloroethane were added over 1 hour to 8,4 g aluminium chloride suspended in 217 g bromobenzene at - 10°C. After stirring for 20 minutes, 204 g aluminium chloride were added, followed by 127 g acetylchloride. The slurry was then stirred at 0°C for 4 hours and at room temperature for 12 hours. The dark mass was then poured into 2 l of ice-water. The organic phase was washed two times with water, dried and evaporated to dryness. Distillation of the residue afforded, 240,6 g 1,1,4,4-tetramethyl-6-acetyl-7-bromo-1,2,3,4-tetrahydronaphthalene, bp_{0.08 Torr}: 148-152° C.
NMR (CDCl₃): 7,5(s, 1H), 7,45(s, 1H), 2,63(s, 3H), 1,67(s, 4H), 1,27(s, 12H)

240,6 g 1,1,4,4-Tetramethyl-6-acetyl-7-bromo-1,2,3,4-tetrahydronaphthalene, 96,7 g ethyleneglycol, 11 g Amberlyst 15 and 120 ml hexane were refluxed over night in flask equipped with a Dean-Stark water trap. Afterwards further 50 g of ethyleneglycol were added and refluxing was continued for 12 hours. The reaction mixture was then diluted with ether and filtered from the catalyst. The solution was then washed with 1 l water containing 2 g sodium carbonate and two times with water, dried and evaporated to dryness. Cristallisation of the residue from hexane afforded 137 g 1,1,4,4-Tetramethyl-6-acetyl-7-bromo-1,2,3,4-tetrahydronaphtalene-ethyleneketal.
NMR (CDCl₃): 7,55(s, 1H), 7,47(s, 1H), 3,97-4,15(m, 2H), 3,69-3,85(m, 2H), 1,82(s, 3H), 1,65(s, 4H), 1,25(s, 12H)

To 1,02 g magnesium-turnings, suspended in 6 ml tetrahydrofurane, were added slowly 310 g ethylbromide. When the Grignard reaction had started, 10,59 g 1,1,4,4-Tetramethyl-6-acetyl-7-bromo-1,2,3,4-tetrahydro-naphthalene-ethyleneketal, dissolved in tetrahydrofurane were added at reflux temperature over a period of several minutes. After refluxing for 2 additional hours, 4,5 g N,N-dimethylformamide were added at 0° C and the mixture was stirred at this temperature for 3 hours. The reaction mixture was then refluxed for 3 hours. The reaction mixture was then poured into 2N HCl and extracted with ether. The organic phase was washed with water, evaporated to dryness and refluxed for 2 hours with 60 ml HCl 2N/ml water. After the usual workup 8,38 g 1,1,4,4-Tetramethyl-6-acetyl-7-formyl-1,2,3,4-tetrahydronaphthalene were obtained. Melting point 116° C: (hexane).
NMR(CDCl₃): 7,84(s, 1H), 7,63(s, +H), 2,63(s, 1H), 1,71(s, 4H), 1,33(s, 12H)

### Example 2

### 1,1,2,4,4-Pentamethyl-6-acetyl-7-formyl-1,2,3,4-tetrahydronaphtalene

This compound was prepared according to Example 1 by acetylation of 1,1,2,4,4-Pentamethyl-7-bromo-1,2,3,4-tetrahydonaphtalene, ketalisation with ethyleneglycol and Grignard-reaction with N-formylmorpholine.
NMR(CDCl₃): 10,2(s, 1H), 7,9(s, 1H), 7,62(s, 1H), 2,64(s, 3H), 1,8-2,0(m, 1H), 1,68(t, 2H), 1,45 (dxd, 1H), 1,38(s, 3H), 1,36(s, 3H), 1,10(s, 3H), 1,02(d, 3H)

### Example 3

### Use of 1,1,4,4-Pentamethyl-6-acetyl-7-formyl-1,2,3,4-tetrahydronaphtalene as fixateur

A muguet-type fragrance composition consisting of

| Ingredient | Parts |
|---|---|
| | |
| Indole | 5 |
| Benzylacetate | 7 |
| cis-3-Hexenyl-acetate (10%) | 7 |
| Rose oxide (1%) | 5 |
| Hedione | 10 |
| Lilial | 20 |
| cis-3-Hexenyl-benzoate | 15 |
| Nerol | 15 |
| cis-3-Hexenol | 10 |
| Cinnamyl alcohol | 30 |
| Citronellyl acetate | 30 |
| Rhodinol | 30 |
| Phenylethanol | 20 |
| Citronellol | 75 |
| Benzyl benzoate | 70 |
| Geraniol | 70 |
| Geranyl acetate | 80 |
| Nerolidol | 80 |
| Farnesol | 120 |
| Benzylic alcohol | 150 |
| Hydroxycitronellal | 50 |
| Dipropylene glycol | 101 |

was prepared. Addition of the title compound resulted in improved linear behaviour of the fragrance composition, e.g. the scent of the fragrance composition was stable over a longer period.

### Example 4

### Use of 1,1,2,4,4-Pentamethyl-6-acetyl-7-formyl-1,2,3,4-tetrahydronaphthalene as fixateur

Addition of the title compound to a muguet-type fragrance composition according to example 3 resulted in improved linear behaviour of the fragrance composition, e.g. the scent of the fragrance composition was stable over a longer period.

## Claims

1. Compounds of formula I 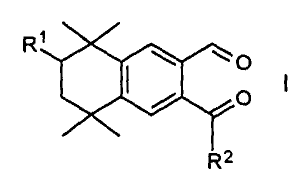 wherein R¹ is hydrogen or methyl, and R² is alkyl.

2. Compounds of formula I according to claim 1, wherein R² is C₁₋₁₂-alkyl, preferably C₁₋₄-alkyl.

3. 1,1,4,4-tetramethyl-6-acetyl-7-formyl-1,2,3,4-tetrahydronaphthalene

4. 1,1,2,4,4-pentamethyl-6-acetyl-7-formyl-1,2,3,4-tetrahydronaphthalene

5. Method for preparing the compounds of formula I 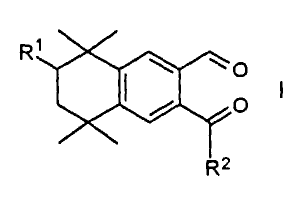 wherein R¹ is hydrogen or methyl and R² is alkyl by reacting a compound of formula II 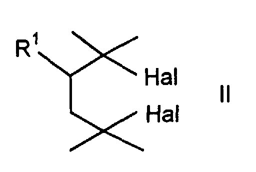 with a compound of formula III 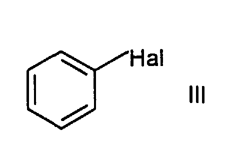 wherein Hal is halogen, acylating the obtained tetrahydronaphtalene of formula IV 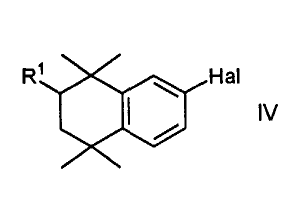 to give a compound of formula V 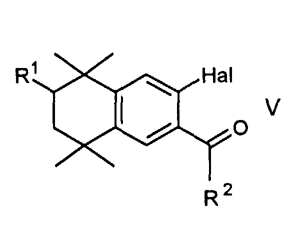 which is reacted with an alcohol or glycol to a ketal of formula VI 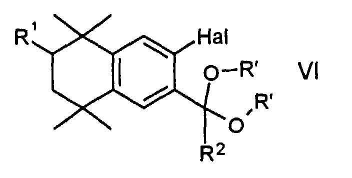 wherein R' is an alkyl residue or R'-R' stands for a divalent alkyl residue, thus forming a dioxolane ring, compound VI is then reacted with magnesium and a formamide of formula VIII 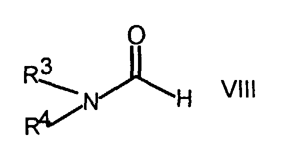 wherein R³ and R⁴ are two organic residues or form together a ring to give a compound of formula I.

6. Method according to claim 5, whereby Hal is selected from the group consisting of chlor and brom.

7. Method according to claim 5 or 6, whereby compounds IV and or VII are not recovered during the process.

8. Method according to one of the claims 5 to 7 whereby the compound of formula VIII is selected from the group consisting of dimethylformamide, N-formylmorpholine and N-methyl-formanilide.

9. Use of the compounds of formula I as fixateur in fragrance compositions.

10. Fragrance composition containing as fixateur one or more compounds of formula I.

11. Fragrance composition according to claim 10 containing 1,1,4,4-tetramethyl-6-acetyl-7-formyl-1,2,3,4-tetrahydronaphthalene as fixateur.

12. Fragrance composition according to claim 10 containing 1,1,2,4,4-tetramethyl-6-acetyl-7-formyl-1,2,3,4-tetrahydronaphthalene as fixateur.

13. Fragrance composition according to one of the claims 10 to 12 comprising highly volatile alcohols.
